# EUROPÄISCHE PATENTSCHRIFT

(11) **EP 2 296 584 B1**
(45) Veröffentlichungstag und Bekanntmachung des Hinweises auf die Patenterteilung: **10.04.2019**
(21) Anmeldenummer: 09776672.9
(22) Anmeldetag: 02.06.2009
(51) Int. Cl.: A61F 2/34, A61F 2/00

(54) **GELENKPFANNE MIT PHYSIOLOGISCHER LASTÜBERTRAGUNG**
SOCKET HAVING PHYSIOLOGICAL LOAD TRANSMISSION
CUPULE COTYLOÏDIENNE AVEC TRANSMISSION PHYSIOLOGIQUE DE LA CHARGE

(30) Priorität: 11.07.2008 DE 102008032705; 12.09.2008 DE 102008047009
(43) Veröffentlichungstag der Anmeldung: 23.03.2011
(73) Patentinhaber: Mathys AG Bettlach, 2544 Bettlach (CH)
(72) Erfinder: DELFOSSE, Daniel, 3306 Jegensdorf (CH); LERF, Reto, 4513 Langendorf (CH)
(74) Vertreter: Körfer, Thomas
(86) Internationale Anmeldenummer: PCT/EP2009/003924
(87) Internationale Veröffentlichungsnummer: WO 2010/003488

(56) Entgegenhaltungen:
- EP-A- 0 761 242
- EP-A- 0 803 234
- EP-A- 1 728 489
- DE-A1- 3 130 732
- DE-A1- 4 227 002
- DE-A1- 4 337 936
- DE-A1- 19 904 436

## Beschreibung

Die Erfindung bezieht sich auf eine Gelenkpfanne für eine Hüftgelenk-Endoprothese zur prothetischen Voll- oder Teilversorgung eines menschlichen oder tierischen Hüftgelenks.

Die europäische Patentanmeldung EP 1 728 489 A1 beschreibt eine Hüftgelenk-Endoprothese mit einer Gelenkpfanne, welche abgeschlossene Bohrungen aufweist. Die einteilige Gelenkpfanne ist zumindest mit einer Schraube in dem Beckenknochen verankert, wobei die Schraube in der Bohrung angeordnet ist.

Die deutsche Offenlegungsschrift DE 43 37 936 A1 beschreibt eine sphärische Hüftgelenkpfanne zum Einsetzen in Knochengewebe mit einer äußeren Metallschale und einer inneren Keramikschale, die in der Metallschale verankert ist.

Aus der deutschen Offenlegungsschrift DE 199 04 436 A1 ist eine Hüftgelenkpfanne mit einem Kopplungselement zwischen dem Pfannengehäuse und dem Pfanneneinsatz bekannt, wobei die Elastizität und die Dämpfungseigenschaft des Kopplungselements durch seine Porösität und durch die Struktur seiner Oberfläche vorgebbar ist.

Die im Stand der Technik beschriebenen Hüft-Endoprothesen haben zwar einen hohen Standard erreicht, wobei eine Überlebensrate von mindestens 90% nach 10 Jahren erreicht, d.h. eine Ausfallsrate von 1% per Jahr nicht überschritten wird. Wie mit Hilfe der skandinavischen Hüftregister nachgewiesen wurde, gibt es sogar Systeme (jeweils die besten zementierten oder unzementierten Hüftschäfte oder Pfannen in ihrer Klasse), die 95% oder mehr Überlebensraten nach 10 Jahren ausweisen. In der zweiten Dekade nach der Implantation nimmt die Ausfallrate jedoch aber drastisch zu. So gibt es heute keine Hüftpfannen, die auch nach 20 Jahren noch eine Überlebensrate von 90% oder mehr aufweisen.

Dazu gibt es vier Gründe:
Nicht alle Materialien, die bis heute bei einem totalen Ersatz des Hüftgelenks eingesetzt werden, garantieren, dass sie "ungealtert", also ohne signifikante Einbusse bezüglich ihrer mechanischen und tribologischen Eigenschaften, über sehr lange Zeiten im Körper verbleiben können.

Nicht alle Materialien, die bis heute als Artikulationspartner bei einem totalen Ersatz des Hüftgelenks Verwendung finden, garantieren, dass die Abriebpartikel sich gutartig über sehr lange Zeiten im Körper verhalten, ohne Gewebeschädigungen hervorzurufen. Nicht alle Materialien, die bis heute an der Oberfläche der vollständigen prothetischen Versorgung des Hüftgelenks eingesetzt wurden, garantieren, dass der das Implantat umgebende Knochen sich langfristig und dauerhaft mit dem Implantat verbindet, also "osseointegriert".

Nicht alle Materialien, die bis heute als Last tragende Struktur der vollständigen prothetischen Versorgung des Hüftgelenks Verwendung finden, garantieren, dass der das Implantat umgebende Knochen sich nicht im Laufe der Zeit durch das so genannte "stress shielding" zurückbildet. Ferner sollte das Dokument DE 42 27 002 A1 als weiterer Stand der Technik nicht unerwähnt bleiben. Dieses beschreibt eine Hüftgelenkpfanne aus zwei Teilen. Der erste Teil umfasst einen Kunststoff-Pfannenkörper und ein auf der konvexen Oberfläche des Kunststoff- Pfannenkörpers angeordnetes metallisches poröses Mantelelement. Dieses metallische poröse Mantelelement besteht aus einer kreisförmigen Scheibe mit einer Porosität von ca. 20 %, die aus Titanpulver auf pulvermetallurgischem Wege durch Sintern hergestellt ist. Durch gemeinsames Erhitzen der tiefgezogenen Scheibe sowie des Kunststoff-Pfannenkörpers und unter Anwendung von Druck wird eine feste mechanische Verbindung zwischen dem Metall der Scheibe und dem Kunststoff des Pfannenkörpers erzielt.

In diesem Rahmen sei auch das Dokument DE 31 30 732 A1 erwähnt, welches ein Endoprothesenteil, beispielsweise eine Hüftgelenkspfanne, offenbart. Dieses Endoprothesenteil weist einen Pfannenkörper auf, der aus einem porösen Keramik-, Porzellan- oder Kohlenstoffmaterial, aus Kunststoff oder Metall besteht, dem durch entsprechende Techniken eine schwammartige Porosität verliehen ist.

Abschließend sei noch auf die Druckschrift EP 0 803 234 A1 hingewiesen, welche eine zweiteilige Hüftgelenkpfanne zeigt, deren Innenschale aus einem metallischen oder keramischen Material besteht und deren äußere Schale entweder aus einem Polymer oder aus Knochenzement aufgebaut ist. Die äußere Oberfläche der äußeren Schale ist mit einem knochenwachstumsfördernden Mittel versetzt, z.B. durch eine poröse Außenschicht oder ein Beschichtung mit Hydroxylapatit.

Der Erfindung liegt die Aufgabe zu Grunde, eine Gelenkpfanne für eine prothetische Versorgung eines menschlichen oder tierischen Hüftgelenks anzugeben, welche auch im zweiten Jahrzehnt nach der Implantation stabil im Körper verankert ist und folgende Bedingungen erfüllt:
Das Material der Gelenkpfanne soll ungealtert über mehr als zwei Dekaden im Körper verbleiben.

Das Material der Gelenkpfanne soll über einen sehr langen Zeitraum abriebbeständig sein.

Das Material an der Oberfläche der Gelenkpfanne soll sich langfristig und stabil mit dem umgebenden Knochenmaterial verbinden.

Das Material der Gelenkpfanne soll die mechanische Last gleichmäßig in das umgebende Knochenmaterial einleiten.

Bezüglich aller oben aufgeführten Bedingungen wird die Aufgabe erfindungsgemäß gelöst durch die Merkmale des Anspruchs 1. Vorteilhafte Weiterbildungen sind Gegenstand der hierauf rückbezogen Unteransprüche.

Anspruch 1 beschreibt eine Gelenkpfanne für eine Hüftgelenk-Endoprothese, wobei die erfindungsgemäße Gelenkpfanne aus alterungsbeständigen Materialien besteht und mit einer Gelenkkugel eine abriebarme Gleitpaarung bildet. Die erfindungsgemäße Gelenkpfanne weist eine Implantatoberfläche auf, welche aus einem Material mit einer porösen Oberfläche gebildet ist. Dabei bewirkt eine isoelastische Struktur der erfindungsgemäßen Gelenkpfanne mit der natürlichen oder der implantierten Gelenkkugel eine physiologische Lastübertragung, wobei speziell konzipierte Materialien zum Einsatz kommen, deren Elastizitätsmodul den Werten eines spongiösen Knochenmaterials angepasst ist.

Ein Vorteil der erfindungsgemäßen Gelenkpfanne besteht darin, dass das Material für die Gelenkpfanne ein Verbundwerkstoff mit einem Elastizitätsmodul bevorzugt zwischen 0,3 GPa und 2,0 GPa, besonders bevorzugt zwischen 0,5 GPa und 1,5 GPa, ist.

Der Verbundwerkstoff der erfindungsgemäßen Gelenkpfanne gewährleistet vorteilhafterweise eine physiologische Lastübertragung auf die umgebenden Knochenstrukturen. Sind die Implantate starrer als der ersetzte Knochen, wird ein großer Teil der Last durch das Implantat aufgenommen und zumeist in einer weit von der Artikulation entfernten Stelle dem umgebenden Knochen wieder übertragen. Ein artikulationsnaher Knochen, wie z.B. der proximale Femur, wird somit zuwenig belastet und atrophiert in der Folge. Nicht geeignet sind alle Materialien mit einem hohen Elastizitätsmodul. Als absolut oberste Grenze gilt 17 GPa, was dem durchschnittlichen Elastizitätsmodul des kortikalen Knochens entspricht. Um aber eine optimale knöcherne Integration der erfindungsgemäßen Gelenkpfanne zu erzielen, darf diese die mechanischen Eigenschaften des Beckens möglichst nicht verändern. Das ideale Material für eine Hüftpfanne ist im Elastizitätsmodul den entsprechenden Werten des spongiösen Knochens (0.3 GPa - 2.0 GPa) angepasst. Damit wird vorteilhafterweise das mechanische und insbesondere das Deformationsverhalten des Beckens nicht beeinflusst.

Vorteilhafterweise wird für die Herstellung der erfindungsgemäßen Gelenkpfanne ein Verbundwerkstoff eingesetzt, so dass die absolut oberste Grenze von 17 GPa im Elastizitätsmodul stets deutlich unterschritten ist.

Des Weiteren ist es günstig, dass der Verbundwerkstoff zu einem bestimmten Anteil aus einem Polymer besteht, welches einfach zu formen ist. Zweckmäßigerweise ist für das Polymer eine Verstärkung mit einem zweiten Material vorgesehen, welches eine Armierung, Fasern oder Partikel sein kann. Das zweite Material ist vorteilhafterweise ein Metall, eine Keramik oder ein zweites Polymer, welches sich gut mit dem ersten Polymer verbindet, bzw. gut in dieses integrierbar ist.

Ferner ist es von Vorteil, wenn die erfindungsgemäße Gelenkpfanne eine Implantatoberfläche mit Partikeln aus Titan oder Kalziumphosphat aufweist. Dadurch ist die Oberfläche zusätzlich vergrößert, was eine gute Osseointegration fördert.

Ein weiterer Vorteil der erfindungsgemäßen Gelenkpfanne besteht bevorzugt in der Herstellung ihrer Oberflächen aus inerten oder bioaktiven Materialien, wie Titan oder Hydroxylapatit. Dadurch ist durch Osseointegration eine dauerhafte feste Verbindung mit dem umgebenden Knochen gewährleistet, wobei die Oberflächen vorteilhafterweise porös oder stark aufgeraut sind.

Ein weiterer Vorteil der erfindungsgemäßen Gelenkpfanne besteht in ihrer bevorzugten Implantatoberfläche aus Titan, Tantal, Kalziumphosphat oder Bioglas, was ebenfalls günstig für die Osseointegration ist.

Ferner ist es von Vorteil, wenn die erfindungsgemäße Gelenkpfanne stabilisiertes und hochvernetztes UHMWPE enthält, welches mittels eines Zusatzes eines Antioxidans alterungsbeständig und oxidationsbeständig ist und nicht versprödet, so dass gewährleistet ist, dass über einen Zeitraum von mehr als zwei Dekaden keine oder nur sehr wenig Abriebpartikel generiert werden.

Vorteilhafterweise ist das Antioxidans Vitamin E, welches vor oder nach Sintern des UHMWPE-Pulvers als Flüssigkeit einfach und gut dosierbar zugesetzt werden kann. Diese Verfahren sind in EP 1 161 489 A1, WO 2004/101009 A1 und WO 2004/064618 A1 beschrieben.

Ferner ist eine aus einem monolytischen Material hergestellte Gelenkpfanne von Vorteil, da für diese Art der Fertigung die Fertigungstoleranzen klein gehalten werden können.

Eine als Verbund aufgebaute Gelenkpfanne, welche eine Implantatoberfläche aufweist, in welche ein Keramik-Inlay für den Anwender untrennbar integriert ist, ist dahingehend von Vorteil, dass die Gleitpaarung zwischen der Gelenkkugel und der Innenseite der erfindungsgemäßen Gelenkpfanne optimiert ist.

Ausführungsbeispiele der vorliegenden Erfindung werden anhand der Zeichnung nachfolgend beschrieben. In der Zeichnung zeigt:
- Fig. 1: eine totale Hüftgelenk-Endoprothese mit der erfindungsgemäßen Gelenkpfanne;
- Fig. 2: eine Schnittdarstellung eines ersten Ausführungsbeispiels der erfindungsgemäßen Gelenkpfanne und
- Fig. 3: eine Schnittdarstellung eines zweiten Ausführungsbeispiels der erfindungsgemäßen Gelenkpfanne.

Einander entsprechende Teile sind in allen Figuren mit den gleichen Bezugszeichen versehen.

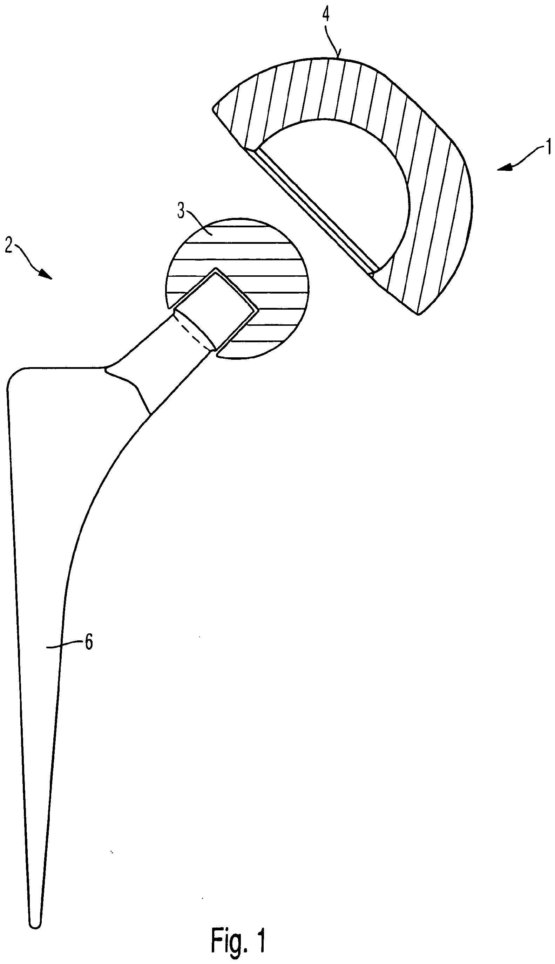

Fig. 1 zeigt eine totale Hüftgelenk-Endoprothese 2 mit der erfindungsgemäßen Gelenkpfanne 1, welche zusammen mit einer Gelenkkugel 3 gemäß der vorliegenden Erfindung eine abriebarme und alterungsbeständige Gleitpaarung bildet, welche auch 20 Jahre nach ihrem Einsetzen noch voll funktionsfähig im menschlichen Körper verbleiben kann. Bei einer totalen Hüftgelenk-Endoprothese 2 ist die Gelenkkugel 3 auf einem proximalen Ende eines Schafts 6 befestigt, welcher in den Knochenmarkkanal eines Femurknochens implantiert wird. Bei einer teilweisen Hüftgelenk-Endoprothese jedoch wird nur die Gelenkpfanne 1 im Knochenmaterial des Beckens verankert.

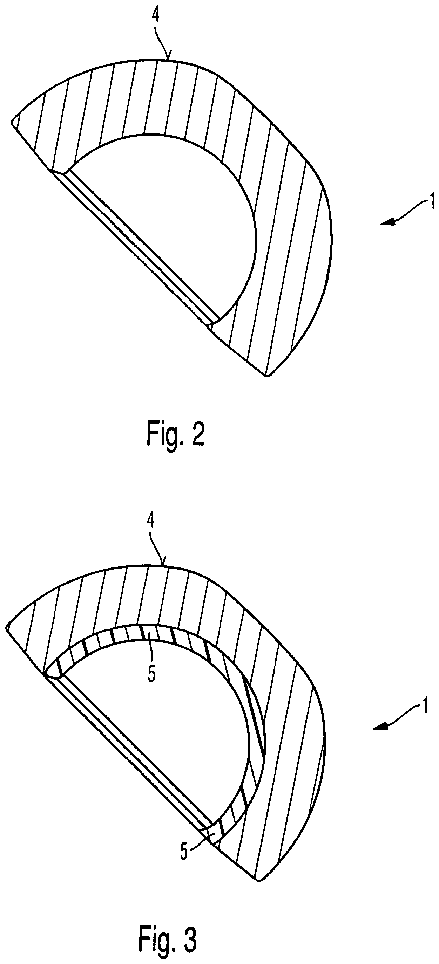

Fig. 2 zeigt ein erstes Ausführungsbeispiel der erfindungsgemäßen Gelenkpfanne 1 für eine Hüftgelenk-Endoprothese 2. Die erfindungsgemäße Gelenkpfanne 1 besteht aus alterungsbeständigen Materialien und bildet mit einer Gelenkkugel 3 eine abriebarme Gleitpaarung, wobei die erfindungsgemäße Gelenkpfanne 3 eine Implantatoberfläche 4 aufweist, welche aus einem Material mit einer porösen Oberfläche gebildet ist. Eine isoelastische Struktur der Gelenkpfanne 1 bewirkt eine physiologische Lastübertragung mittels speziell konzipierter Materialien, indem deren Elastizitätsmodul erfindungsgemäß den Werten eines spongiösen Knochenmaterials angepasst ist.

Das in Fig. 2 gezeigte erste Ausführungsbeispiel der erfindungsgemäßen Gelenkpfanne 1 bezieht sich auf eine Herstellung aus einem einzigen monolytischen Material, wodurch eine präzise und kostengünstige Fertigung der erfindungsgemäßen Gelenkpfanne 1 mittels Fräsen oder Drehen aus einem einzigen Werkstoffblock möglich ist. Die isoelastische Struktur der erfindungsgemäßen Gelenkpfanne 1 ist dann gegeben, wenn deren mechanische Eigenschaften wie Festigkeit und Belastbarkeit den mechanischen Eigenschaften des Knochenmaterials entsprechen, in welches die Gelenkpfanne implantiert wird.

Das Material für die erfindungsgemäße Gelenkpfanne 1 ist ein Verbundwerkstoff mit einem Elastizitätsmodul zwischen 0,3 GPa und 2,0 GPa, wobei der Verbundwerkstoff, der zu einem bestimmten Anteil aus einem Polymer besteht, bevorzugt ein Elastizitätsmodul im Bereich von 0,5 GPa bis 1,5 GPa und besonders bevorzugt im Bereich von 0,8 GPa bis 1,2 GPa aufweist.

Für das Polymer des Verbundwerkstoffs kann eine Verstärkung mit einem zweiten Material vorgesehen werden, wobei als Verstärkung Armierungen, Fasern oder Partikel vorgesehen sind, welche aus einem Metall, einer Keramik, aus Graphit oder aus einem zweiten Polymer mit höherer Festigkeit bestehen.

Die erfindungsgemäße Gelenkpfanne 1 weist eine strukturierte Implantatoberfläche 4 aus inerten oder bioaktiven Materialien, wie Titan, Tantal, Kalziumphosphat, Hydroxylapatit oder Bioglass auf, wobei das stabilisierte UHMWPE aufgrund eines Zusatzes von einem Antioxidans, wie beispielsweise Vitamin E, verbesserte Materialeigenschaften wie eine erhöhte Alterungsbeständigkeit und Oxidationsbeständigkeit aufweist. Dabei wird das Vitamin E vor oder nach dem Sintern des UHMWPE-Pulvers zum Festkörper zugeführt.

Die Implantatoberfläche 4 der erfindungsgemäßen Gelenkpfanne 1 kann aus porösen Titan, Tantal, Kalziumphosphat oder Bioglas bestehen oder aus einzelnen Partikel aus Titan oder Kalziumphosphat bestehen, welche zusätzlich die Osseointegration in das Knochenmaterial verbessern ohne die Stufigkeit des Verbundes wesentlich zu erhöhen.

Fig. 3 zeigt ein zweites Ausführungsbeispiel der erfindungsgemäßen Gelenkpfanne 1 mit einem modularen Aufbau, wobei in der Implantatoberfläche der erfindungsgemäßen Gelenkpfanne 1 ein Keramik-Inlay 5 integriert ist, welches mit der physiologischen oder der implantierten Gelenkkugel 3 eine altersbeständige und sehr abriebsarme Gleitpaarung bildet.

Das Keramik-Inlay 5 ist derart gestaltet, dass es die Elasitzität der Pfanne (z.B. gemessen als radiale Stufigkeit über die äußere Äquator-Linie) nicht wesentlich verringert. Andererseits darf die Wanddicke des Keramik-Inlays 5 einen bestimmten Mindestwert nicht unterschreiten, um die Bruchsicherheit zu gewährleisten. Die Dicke liegt je nach Keramik (bevorzugt wird eine Dispersionskeramik verwendet) bei rund 2 bis 4 mm.

Die Erfindung ist nicht auf die in der Zeichnung dargestellten Ausführungsbeispiele, insbesondere nicht auf eine prothetische Vollversorgung eines menschlichen oder tierischen Hüftgelenks, beschränkt. Eine Anwendung der erfindungsgemäßen Gelenkpfanne 1 in einer Schulterprothese ist ebenfalls vorgesehen.

## Patentansprüche

1. Gelenkpfanne (1) für eine Hüftgelenk-Endoprothese (2),
wobei die Gelenkpfanne (1) eine poröse Implantatoberfläche (4) zur Verbindung mit umgebendem Knochenmaterial aufweist, und
wobei die Gelenkpfanne (1) aus einem monolithischen Material hergestellt ist, dieses Material ein Verbundwerkstoff ist, und der Verbundwerkstoff ein Polymer sowie eine Verstärkung mit einem Metall, einer Keramik, einem Graphit oder einem zweiten Polymer mit höherer Festigkeit umfasst, um ein Elastizitätsmodul des Materials den Werten eines spongiösen Knochenmaterials anzupassen,
**dadurch gekennzeichnet, dass** die Gelenkpfanne (1) vorgesehen ist, um über ein Keramik-Inlay (5) mit einer natürlichen oder künstlichen Gelenkkugel (3) eine Gleitpaarung zu bilden,
**und dadurch,** dass die Implantatoberfläche (4) aus einzelnen Partikeln aus Titan oder Kalziumphosphat besteht.

2. Gelenkpfanne nach Anspruch 1,
**dadurch gekennzeichnet,**
**dass** der Verbundwerkstoff ein Elastizitätsmodul zwischen 0,3 GPa und 2,0 GPa, bevorzugt zwischen 0,5 GPa und 1,5 GPa, aufweist.

3. Gelenkpfanne nach Anspruch 2,
**dadurch gekennzeichnet,**
**dass** der Verbundwerkstoff für die Gelenkpfanne (1) bevorzugt ein Elastizitätsmodul im Bereich von 0,5 GPa bis 1,5 GPa und besonders bevorzugt im Bereich von 0,8 GPa bis 1,2 GPa aufweist.

4. Gelenkpfanne nach einem der vorhergehenden Ansprüche,
**dadurch gekennzeichnet,**
**dass** die Verstärkung des Verbundwerkstoffs in Form von Armierungen, Fasern oder Partikeln vorliegt.

5. Gelenkpfanne nach einem der Ansprüche 1 bis 4,
**dadurch gekennzeichnet,**
**dass** Gelenkpfanne stabilisiertes und hochvernetztes UHMWPE enthält.

6. Gelenkpfanne nach Anspruch 5,
**dadurch gekennzeichnet,**
**dass** das stabilisierte UHMWPE mittels eines Zusatzes eines Antioxidans alterungsbeständig und oxidationsbeständig ist.

7. Gelenkpfanne nach Anspruch 6,
**dadurch gekennzeichnet,**
**dass** das dem UHMWPE zugeführte Antioxidans Vitamin E ist.

8. Gelenkpfanne nach Anspruch 7,
**dadurch gekennzeichnet,**
**dass** pulverförmiges Vitamin E vor oder während oder nach dem Sintern des UHMWPE-Pulvers zugeführt ist.

9. Gelenkpfanne nach einem der Ansprüche 1 bis 8,
**gekennzeichnet durch**
ihren modularen Aufbau aus der Implantatoberfläche (4), in welche das Keramik-Inlay (5) integriert ist.

## Claims

1. A socket (1) for a hip joint endoprosthesis (2),
wherein the socket (1) has a porous implant surface (4) for bonding to surrounding bone material, and
wherein the socket (1) is made from monolithic material, said material is a composite material and the composite material comprises a polymer as well as a reinforcement including a metal, a ceramic, a graphite or a second polymer of higher strength to adapt an elasticity modulus of the material to the values of a spongy bone material,
**characterized in that** the socket (1) is provided to form a sliding pairing with a natural or artificial joint ball (3) via a ceramic inlay (5),
**and in that** the implant surface (4) is made from individual particles of titanium or calcium phosphate.

2. The socket according to claim 1,
**characterized in that**
the composite material has a modulus of elasticity ranging from 0.3 GPa to 2.0 GPa, of preference from 0.5 GPa to 1.5 GPa.

3. The socket according to claim 1,
**characterized in that**
the composite material for the socket (1) preferably has a modulus of elasticity ranging from 0.5 GPa to 1.5 GPa and especially preferred ranging from 0.8 GPa to 1.2 GPa.

4. The socket according to any one of the preceding claims,
**characterized in that**
the reinforcement of the composite material is present in the form of strengthening elements, fibers or particles.

5. The socket according to any one of the claims 1 to 4,
**characterized in that**
the socket contains stabilized and highly cross-linked UHMWPE.

6. The socket according to claim 5,
**characterized in that**
the stabilized UHMWPE is resistant to ageing and resistant to oxidation by means of addition of an antioxidant.

7. The socket according to claim 6,
**characterized in that**
the antioxidant fed to the UHMWPE is vitamin E.

8. The socket according to claim 7,
**characterized in that**
the powdered vitamin E is fed before or during or after sintering of the UHMWPE powder.

9. The socket according to any one of the claims 1 to 8,
**characterized by**
the modular structure thereof from the implant surface (4) in which the ceramic inlay (5) is integrated.

## Revendications

1. Cupule cotyloïdienne (1) pour une endoprothèse de la hanche (2),
dans laquelle la cupule cotyloïdienne (1) présente une surface d'implant (4) poreuse pour la liaison avec de la matière osseuse environnante, et
dans laquelle la cupule cotyloïdienne (1) est fabriquée à partir d'une matière monolithique, cette matière est un matériau composite, et le matériau composite comprend un polymère et un renforcement avec un métal, une céramique, un graphite ou un deuxième polymère à résistance plus élevée pour adapter un module d'élasticité de la matière aux valeurs d'une matière osseuse spongieuse,
**caractérisée en ce que** la cupule cotyloïdienne (1) est destinée à former un appariement de surfaces de glissement avec une rotule articulaire (3) naturelle ou artificielle au-dessus d'un inlay en céramique (5),
et **caractérisée en ce que** la surface d'implant (4) est constituée de différentes particules de titane ou de phosphate de calcium.

2. Cupule cotyloïdienne selon la revendication 1,
**caractérisée**
**en ce que** le matériau composite présente un module d'élasticité entre 0,3 GPa et 2,0 GPa, de préférence entre 0,5 GPa et 1,5 GPa.

3. Cupule cotyloïdienne selon la revendication 2,
**caractérisée**
**en ce que** le matériau composite pour la cupule cotyloïdienne (1) présente un module d'élasticité dans la plage de 0,5 GPa à 1,5 GPa et de manière particulièrement préférée entre 0,8 GPa à 1,2 GPa.

4. Cupule cotyloïdienne selon l'une des revendications précédentes,
**caractérisée**
**en ce que** le renforcement du matériau composite se présente sous la forme d'armatures, de fibres ou de particules.

5. Cupule cotyloïdienne selon l'une des revendications 1 à 4,
**caractérisée**
**en ce que** la cupule cotyloïdienne comprend un UHMWPE stabilisé et hautement réticulé.

6. Cupule cotyloïdienne selon la revendication 5,
**caractérisée**
**en ce que** l'UHMWPE stabilisé résiste au vieillissement et à l'oxydation au moyen d'un ajout d'antioxydant.

7. Cupule cotyloïdienne selon la revendication 6,
**caractérisée**
**en ce que** l'antioxydant ajouté à l'UHMWPE est la vitamine E.

8. Cupule cotyloïdienne selon la revendication 7,
**caractérisée**
**en ce que** de la vitamine E pulvérulente est additionnée avant ou pendant ou après le frittage de la poudre d'UHMWPE.

9. Cupule cotyloïdienne selon l'une des revendications 1 à 8,
**caractérisée par**
sa structure modulaire constituée de la surface d'implant (4), dans laquelle est intégré l'inlay en céramique (5).
